# EUROPEAN PATENT APPLICATION

(11) **EP 4 056 671 A1**
(43) Date of publication of application: **14.09.2022**
(21) Application number: 21161867.3
(22) Date of filing: 10.03.2021
(51) Int. Cl.: C12M 1/00, C12M 1/26

(54) **METHOD FOR OPERATING A BIOPROCESS INSTALLATION**

(71) Applicant: Sartorius Stedim Biotech GmbH, 37079 Göttingen (DE)
(72) Inventor: Saballus, Martin, 37249 Neu-Eichenbert (DE); Reger, Lucas Nik, 37073 Göttingen (DE); Kampmann, Markus, 44149 Dortmund (DE); Matuszczyk, Jens-Christoph, 37079 Göttingen (DE)
(74) Representative: Gottschald Patentanwälte Partnerschaft mbB

(57) **Abstract**

The invention is directed to a method for operating a bioprocess installation (1) with an electronic process control (2) and at least one bioprocess unit (3), wherein the bioprocess unit (3) comprises a cell broth source with a first receptacle (4) for cell broth including cultivation media and cells, establishing a culture environment (A, B) for cell cultivation and/or bio production, wherein the bioprocess unit (3) comprises a clarification setup (5) with a centrifuge (6) for the clarification of the cell broth by centrifugation, with a liquid pumping arrangement (7) assigned to the centrifuge (6) and with a liquid network (8) with a number of liquid lines (9) communicating with the liquid pumping arrangement (7), wherein out of a first culture environment (A) established by the first receptacle (4), the cell broth is transferred to the centrifuge (6) via the liquid network (8), which centrifuge (6) is operated in a forward operation for cell separation and/or cell washing and in a backward operation for cell discharging. It is proposed that the liquid network (8) comprises a recycling line (15) and that in the backward operation, at least part of the discharged cells are being transferred into a second culture environment (B) different from the first culture environment (A) via the recycling line (15) for subsequent cell cultivation and/or bioproduction.

## Description

The present invention relates to a method for operating a bioprocess installation according to the general portion of claim 1, a bioprocess installation with an electronic process control and at least one bioprocess unit according to claim 17, an electronic process control of the bioprocess installation according to claim 20 and use of a pre-mounted liquid network product according to claim 21.

The term "bioprocess" presently represents any kind of biotechnological processes, in particular biopharmaceutical processes. An example for such bioprocess is the use of a bioreactor to cultivate microorganisms or mammalian cells under given conditions, wherein a cell broth is transferred from the bioreactor to a downstream process.

The method in question for operating a bioprocess installation may be applied in various fields of biotechnology. High efficiency in this field has been driven by the increasing demand for biopharmaceutical drugs. The efficiency in this sense is regarding not only the cost-effectiveness of the components to be used but also the controllability of the processes connected thereto. The method in question serves for establishing a culture environment for cell cultivation and/or bioproduction, followed by clarification of a cell broth by centrifugation and subsequent transfer of discharged cells to a second culture environment via a recycling line for subsequent cell cultivation and/or bioproduction. Possible products can be for instance biopharmaceutical products, in particular proteins, such as growth factors, hormones, enzymes, antibodies, antibody derivates, or the like.

The known method for operating a bioprocess installation (EP 2 310 486 B1), which is the starting point for the invention, makes use of a centrifuge with a number of centrifuge chambers, which are each assigned a chamber inlet and a chamber outlet. The method for operating a bioprocess installation makes also use of a liquid pumping arrangement assigned to the centrifuge and a liquid network for the transport of the liquid, which is based on the cell broth to be clarified. After the clarification of a part of the cells from a bioreactor, the cells can be re-transferred into the same bioreactor leading to a backmixing with the retained cells and media, including dissolved substances, e.g. toxins. Finally, the method for operating a bioprocess installation makes use of a process control for controlling at least the centrifuge and the liquid pumping arrangement. The known method as such is running in a highly efficient manner. However, the resulting combination is of restricted efficiency in view of product yield, process flexibility and adjustability in the mechanical setup as well as in the controllability of the overall process.

It is an object of the present invention to provide a method for operating a bioprocess installation, which increases the bioprocess efficiency and productivity, with as little effort as possible.

The above-noted problem is solved for a method for operating a bioprocess installation with the features of the general part of claim 1 by the features of the characterizing part of claim 1 .

The general concept underlying the invention is based on a transfer of at least a part of the discharged cells to a second culture environment, that is different from the first culture environment.

The term "culture environment" presently means the complex influence of the entirety of all physical, chemical and/or biological parameters, such as temperature, pH and nutrition concentrations, that act upon a cultivated organism and ultimately determine its physiology and survival.

The above is facilitated by a liquid network comprising a recycling line and by a backward operation of a centrifuge. In the second culture environment, which is different from the first culture environment, the recycled cells can be used for subsequent cell cultivation and/or bioproduction. The second culture environment, being different from the first culture environment, gives the basis for an improvement of the subsequent culture environment. First of all, this enables to influence, preferably to optimize, the culture environment leading to influenced, preferably optimized and/or accelerated, cultivation of recycled cells and/or bioproduction by these recycled cells. Second, this allows for elongated cultivation by recycling harvested viable cells, since no seed train needs to be generated for a subsequent bioprocess. Third, this setup allows for an entire removal of consumed media, preventing a backmixing of consumed media with fresh media, including cellular produced inhibitors. This means, that no continuous media exchange is required during cultivation, leading to a reduced media consumption, higher product concentrations, as well as increased productivity, which is not only cost-efficient but also enables a simplified and more efficient control of the overall process. Eventually, this leads to reduced process times and cultivation costs with minimal effort, leading to an increased bioprocess efficiency. Furthermore, this simplified setup allows for an adaption to existing bioprocesses, independent from the process platforms and cell lines used in the respective bioprocess.

In detail, it is proposed, that the liquid network comprises a recycling line and that in the backward operation, at least part of the discharged cells is being transferred to a second culture environment different from the first culture environment via the recycling line for subsequent cell cultivation and/or bioproduction.

The term "recycling line" relates to a liquid line designed for the re-transfer and hence re-use of discharged cells, thus making the cells accessible for subsequent cell cultivation and/or bioproduction.

The preferred embodiment according to claim 2 refers to the possible differences between the first and second culture environment and enables for an increased flexibility regarding the choice of the respective entity, liquid properties and/or culture environment conditions of the first and second culture environment.

According to claim 3, the second culture environment preferably provides culture environment conditions favoring cell growth and/or bioproduction, wherein, alternatively or in addition, the second culture environment is the first culture environment, after having (completely) exchanged the cultivation media. All of this allows for a boost in bioproduction. A complete exchange of the cultivation media, preferably without a backmixing of consumed media and fresh media, favors cell growth and/or bioproduction in the second culture environment.

Claims 4 to 6 are directed to preferred embodiments of the involved receptacles, which allow for an increased flexibility regarding the choice of the second receptacle.

The preferred embodiment according to claim 7 is directed to the transfer of the cell broth from the second receptacle to the first receptacle after the cells have been nourished with fresh media in the centrifugation chamber and transferred to an intermediate phase. This allows the cells to be released from any spent media and toxic by-products before they are used for subsequent cell cultivation and/or bioproduction.

According to claim 8, a termination condition has to be fulfilled with respect to the liquid level in the first receptacle, before the cell broth is transferred from the second to the first receptacle. This is another measure to prevent backmixing of consumed and fresh media.

The preferred embodiment according to claim 9 is directed to the bioprocess units being cascaded. This cascade offers the advantage that allows a continuous recycling of cells and their re-use in a subsequent bioprocess.

According to claim 10, a startup phase is initiated in the second culture environment in order to establish conditions for cell cultivation and/or bioproduction. This leads to an increased ratio of viable cells and hence to an increased productivity of the bioprocess.

In claims 11 and 12, the properties of the cell washing step and cell discharging step are specified. These specifications allow for an optimized preparation of the cells during forward or backward operation of the centrifuge, as well as for optimized conditions for a subsequent cell cultivation and/or bioproduction.

The preferred embodiment according to claim 13 is directed towards preferred options for automation by providing a valve arrangement. The valve arrangement serves for activating/deactivating the liquid lines of the liquid network in order to support the execution of the respective task.

Claim 14 defines, that the centrifuge and/or the liquid pumping arrangement and/or the valve arrangement are preferably controlled by the electronic process control. The electronic process control preferably operates on a control software, which makes operation particularly flexible.

According to claim 15, the first and/or second receptacle are provided as bioreactor(s). Here it shows that the proposed solution is applicable for a vast number of different process setups.

Claim 16 is directed to a sensor arrangement comprising a biomass sensor in the recycling line. The sensor data of the biomass sensor define, when and where the liquid is transferred by the electronic process control to the second culture environment.

A second independent teaching according to claim 17 is directed to a bioprocess installation with an electronic process control and at least one bioprocess unit. The liquid network of the bioprocess installation comprises a recycling line enabling for a transfer of discharged cells into a second culture environment. All explanations given with regard to the first teaching are fully applicable to this second teaching.

According to claim 18, the electronic process control is preferably able to perform the method by controlling the centrifuge and/or the liquid pumping arrangement and/or the valve arrangement, while claim 19 is directed to centrifuge specifications, enabling for an efficient design of the centrifuge.

A third independent teaching according to claim 20 is directed to an electronic process control of the proposed bioprocess installation. The electronic process control is designed to perform the proposed method by controlling the centrifuge and/or the liquid pumping arrangement and/or the valve arrangement. All explanations given with regard to the first and second teachings are fully applicable to this third teaching.

A fourth independent teaching according to claim 21 is directed to the use of a pre-mounted liquid network product, comprising a structure of interconnected liquid lines, as at least part of the liquid network of the proposed bioprocess installation. The pre-mounting of the liquid network makes it particularly easy to quickly enable the bioprocess installation for operation. Moreover, the resulting exchangeability of the liquid network product as a whole makes it easy to provide sterile conditions. This is particularly interesting for the recycling line in view of ensuring predetermined conditions for the subsequent cell cultivation (claim 22).

According to claim 23, the structure of the interconnected liquid lines of the pre-mounted liquid network product can preferably be designed as a single-use component, hence guaranteeing a cost-efficient process and at the same time perfectly sterile conditions. Again, all explanations given with regard to the first, second and third teaching are fully applicable to this fourth teaching.

In the following, a preferred embodiment of the invention is being described with regard to the drawings. In the drawings
- Fig. 1: schematically shows a first exemplary embodiment of a proposed bioprocess installation, with which a proposed method is executable,
- Fig. 2: schematically shows a second exemplary embodiment of a proposed bioprocess installation, with which a proposed method is executable,
- Fig. 3: schematically shows a third exemplary embodiment of a proposed bioprocess installation, with which a proposed method is executable and
- Fig. 4: schematically shows an enlarged view of a proposed clarification setup of the proposed bioprocess installation according to Fig. 1 to 3,
- Fig. 5: shows the results of the proof of concept experiments, in which the proposed method was executed.

The proposed method for operating a bioprocess installation 1 is preferably assigned to the upstream and downstream processes of a bioprocess, processing a liquid in the form of a cell broth for cell cultivation and/or bioproduction.

The term "liquid" is to be understood in a broad sense. It includes not only a pure liquid as such, but also emulsions and suspensions, e.g. a heterogeneous mixture of at least two different liquids or a heterogeneous mixture of solid particles and liquid.

The term "cell broth" is a suspension of cells and/or cell debris in media and describes the entirety of cultivation medium and the respective organism cultured in the cultivation medium. Accordingly, the term "cell broth source" means any manufactured device or system capable of producing and/or storing cell broth.

The term "upstream process" involves all the steps related to cell bank, inoculum (seed train) development, media development, optimization of growth kinetics and the cultivation process itself as well as the corresponding *in-process* control. The harvest of cells can be seen as both, part of upstream- and part of downstream-processing.

The term "downstream process" involves all the steps related to the recovery and the purification of biosynthetic products, particularly biopharmaceuticals, from natural sources such as animal or plant tissue or cell broth, including the recycling of salvageable components and the proper treatment and disposal of waste.

In general, cultivation of cells is currently used for the production of biopharmaceuticals, in particular proteins, such as human insulin, growth factors, hormones, vaccines, or antibodies, antibody derivates, or the like. The products may as well be non-biopharmaceuticals, such as enzymes for food processing, laundry detergent enzymes, biodegradable plastics or biofuels. The focus of the present invention is on biopharmaceutical products secreted by the cells into the supernatant, such as antibodies or exosomes. Additionally or alternatively, the product can be the cells themselves, in particular mammalian cells including stem cells or immune cells such as CAR-T cells for the treatment of cancer.

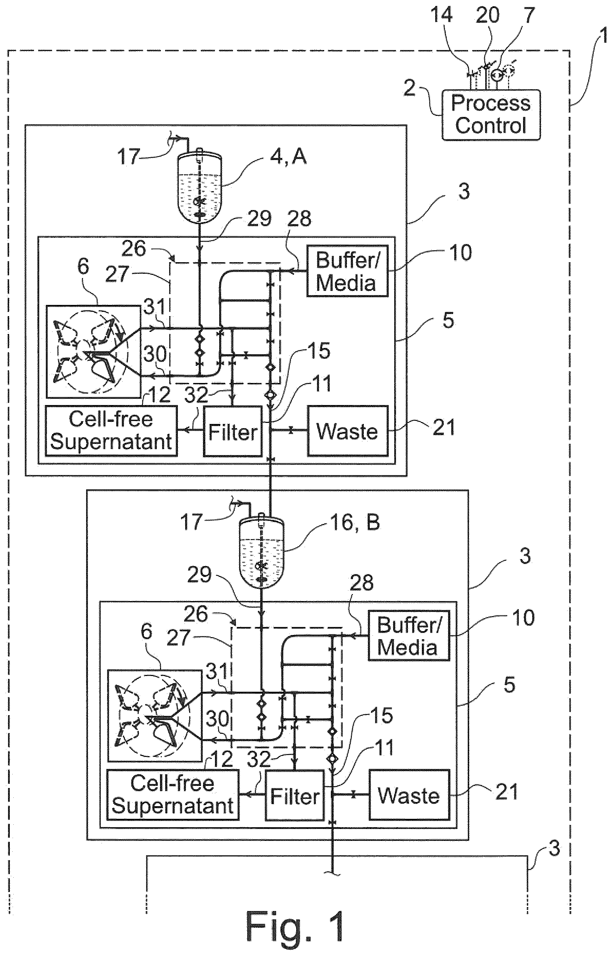

As shown in Fig. 1 to 3, according to all embodiments the proposed method for operating a bioprocess installation 1 employs an electronic process control 2 and at least one bioprocess unit 3. The bioprocess unit 3 comprises a cell broth source with a first receptacle 4 for cell broth including cultivation media and cells. The term "cultivation media" means that the, preferably microbial or eukaryotic, cells used for the bioprocess grow in specially designed growth media, which supply the nutrients required by the respective organisms or cells. A variety of media exist, but invariably contain at least a carbon source, a nitrogen source, water, salts, and micronutrients.

Here and preferably, the respective culture environment A, B for cell cultivation and/or bioproduction is established by at least one physical, chemical and/or biological parameter, such as temperature, pH, carbon source concentration, nitrogen source concentration, amino acid source concentration, oxygen concentration, oxygen uptake rate, carbon dioxide concentration, media composition, type of media, type of receptacle, type of particles, in particular cells, cell concentration, cell viability, cell growth rate, cell productivity and/or biomass production rate.

Moreover, the bioprocess unit 3 comprises a clarification setup 5. This clarification setup 5 carries out a physical process using gravity to remove suspended solids from a liquid phase. In general, the proposed clarification setup 5 can be used to separate any solid/liquid components from each other, including but not limited to cells and media.

For centrifugation, the clarification setup 5 comprises a centrifuge 6 for the clarification of the cell broth by centrifugation. "Centrifugation" is a term for sedimentation of particles in an artificially, by centrifugal forces created, gravitational field, wherein a significant reduction of separation time is achieved via large accelerating forces.

Here and preferably, the centrifuge 6 is designed as a fluidized bed centrifuge for performing a continuous centrifugation process. Preferred setups of the centrifuge 6 are described in EP 2 485 846 A1, the contents of which are hereby incorporated by reference herein.

The clarification setup 5 comprises a liquid pumping arrangement 7 assigned to the centrifuge 6 and a liquid network 8 with a number of liquid lines 9 communicating with the liquid pumping arrangement 7. Out of a first culture environment A established by the first receptacle 4, the cell broth is transferred to the centrifuge 6 via the liquid network 8.

The centrifuge 6 comprises a rotor, which may be rotated around the centrifuge rotor axis by a preferably electric motor. For centrifugation, the liquid pumping arrangement 7 pumps cell broth to the centrifuge 6.

The centrifuge revolution speed, as well as the pumping rate, are, here and preferably, adjustable by the electronic process control 2, with the aim to establish a fluidized bed of particles, such as cells or cell debris, in the centrifuge 6. A fluidized bed is achieved when the centrifugal force on a particle is equal to the opposing fluid flow force so that a zero net force is exerted on the particle.

The centrifuge 6 can be operated in a forward operation for cell separation and/or cell washing. "Forward operation" means one out of two possible fluid flow directions in a centrifuge and describes the operation leading to a separation of liquid and solid particles, such as media and cells. This "forward operation" allows, on the one hand, a washing of separated cells with buffer, preferably PBS buffer, or media, preferably cultivation media, further preferably enriched media, coming from the buffer/media receptacle 10, and/or, on the other hand, the clarification of the supernatant. The goal here is to clarify the liquid supernatant from solid particles such as cells, cell debris, etc., which solid particles are considered biomass. The product to be obtained in this forward operation is the supernatant of the cell broth containing a product of interest, e.g. a recombinant protein, in particular an antibody.

The term "enriched media" describes media that comprise higher concentrations of vitamins, growth factors, carbon-source, nitrogen-source and/or amino acid concentrations or the like and, preferably, allow the respective organism to grow at its maximum growth rate due to the optimized nutrient concentrations. Growth factors and trace nutrients are included in the media for organisms incapable of producing all of the vitamins they require. Inorganic nutrients, including trace elements such as iron, zinc, copper, manganese, molybdenum and cobalt are typically present in unrefined carbon and nitrogen sources but may have to be added when purified carbon and nitrogen sources are used.

In order to obtain a preferably particle-free, further preferably cell-free, supernatant via forward operation of the centrifuge 6, the centrifuged cell broth is subsequently pumped through a filter arrangement 11 of the clarification setup 5. Connected to the filter arrangement 11 is a supernatant reception 12, in particular a supernatant vessel. It is generally possible, that the at least one filter of the filter arrangement 11 is activated and deactivated manually, for example by at least one manually controllable valve 13.

It is also possible, that no valve 13 is assigned to the filter arrangement 11, in which case, instead of deactivating part of the filter arrangement 11, the flushing of the filter arrangement 11 is being started manually. The term "flushing" means a pre-conditioning of filters by rinsing them with buffer before the centrifugation process is started. In addition, after the centrifugation process is finished, a post-flushing of the filters is also preferred in some cases in order to flush out remaining product in the filters and filter lines into the supernatant reception 12.

Alternatively, the centrifuge 6 can be operated in a backward operation. "Backward operation" means the second out of two possible fluid flow directions in a centrifuge and describes the operation leading to a discharge of the separated solid particles, preferably cells. The product to be obtained in backward operation are the cells in the cell broth.

It is particularly essential for the invention, that the liquid network 8 comprises a recycling line 15. In backward operation of the centrifuge 6, at least part of the discharged cells is being transferred to a second culture environment B via the recycling line 15.

This second culture environment B is different from the first culture environment A and set up for a recovery of the discharged cells. Subsequently, the discharged cells are used for cell cultivation and/or bioproduction. The term "recovery" is to be understood in a broad sense. It includes not only the collection of the discharged cells and their concentration in one particular place but is also providing an environment that maintains, feeds and/or nourishes the discharged cells. These measures prepare the discharged cells for subsequent cell cultivation and/or bioproduction. The term "recovery" according to the present teaching may also refer to the clarification and separation of the product of interest (e.g. an antibody) in the supernatant from the cell broth.

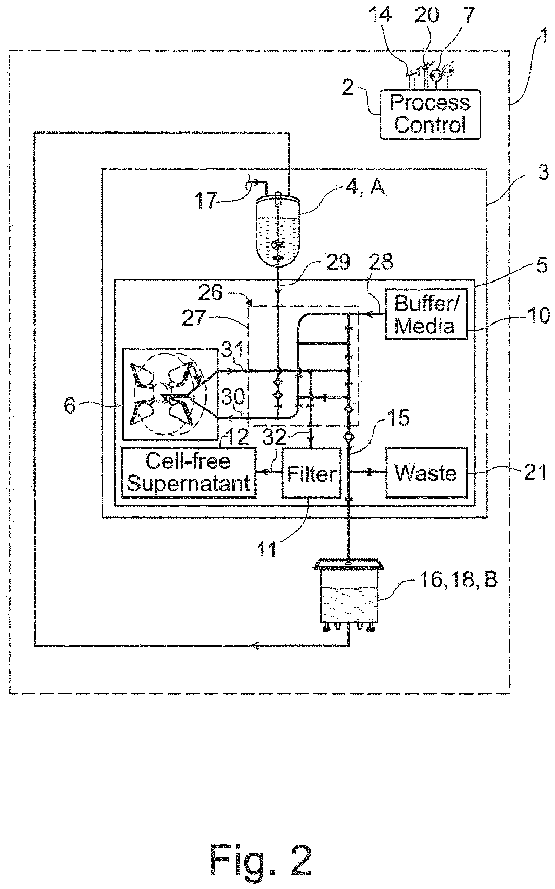

In the embodiments according to Fig. 1 and 2, here and preferably, the second culture environment B differs from the first culture environment A with respect to the structural entity establishing the respective culture environment A, B. The entity establishing the second culture environment B can, for instance, be a second storage vessel or production vessel, such as a second bioreactor.

Additionally or alternatively, and as is provided by all embodiments in Fig. 1 to 3, the second culture environment B differs from the first culture environment A with respect to liquid properties, e.g. choice of media, in particular choice of enriched media, comprising i.a. optimized carbon-source concentrations, optimized nitrogen-source concentrations, optimized amino acid concentrations and/or optimized growth factor concentrations, and/or the liquid volume used in the bioprocess.

Further additionally or alternatively, and as is also provided by all embodiments in Fig. 1 to 3, the second culture environment B differs from the first culture environment A with respect to culture environment conditions, such as choice of gas concentrations, in particular, oxygen and/or carbon dioxide concentrations, and/or choice of pH, and/or choice of temperature or the like. Exemplary amino acid-sources can be peptone or tryptone in concentrations of 0.5 % to 2 %. Exemplary carbon-sources can be glucose or sucrose in concentrations of 0.1 % to 3 %.

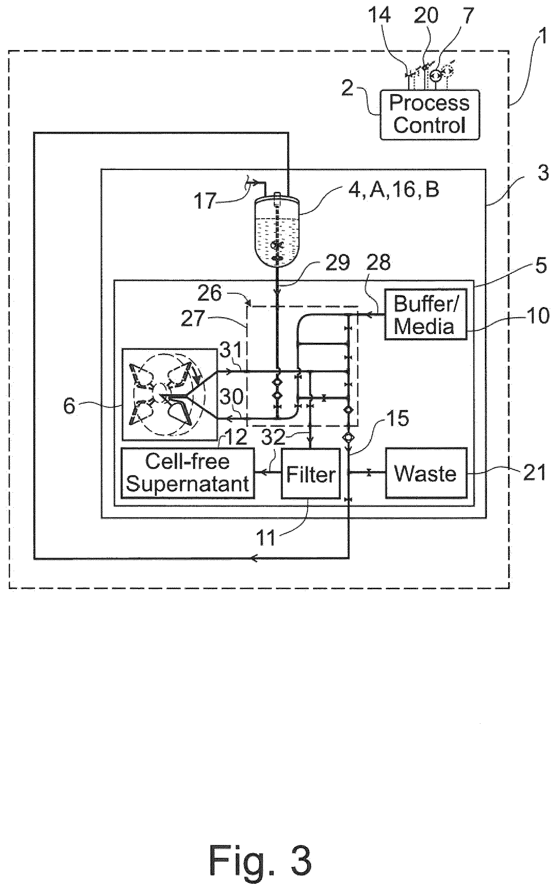

In the embodiment of Fig. 1, the further embodiment of Fig. 2, as well as in the yet further embodiment of Fig. 3, here and preferably, the second culture environment B is the first culture environment A, after having, preferably completely, exchanged the cultivation media. In these embodiments, the discharged cells can be transferred for re-use of the cells for cell cultivation and/or bioproduction, after the cultivation media and/or cells have been, at least partially, removed. Thereby a backmixing of consumed and fresh cultivation media and/or cells is individually controllable.

Preferably, the resulting ratio of consumed to fresh liquid is individually adjustable. Additionally or alternatively, the second culture environment B preferably provides culture environment conditions thereby favoring cell growth and/or bioproduction. The term "Favoring cell growth and/or bioproduction" means an enhancement and support of at least one parameter either mirroring the cells' vitality and/or the cells' productivity, including but not limited to growth rate, viability (i.e. percentage of living cells), productivity, oxygen uptake rate and/or biomass production rate.

As shown in Fig. 3, in principle, the second culture environment B may be provided by the first receptacle 4, which, for establishing the second culture environment B, is being brought to a cell-free and/or a liquid-free state in a preparation phase. In a preferred embodiment, the preparation phase consists of at least one workflow step to establish conditions for cell cultivation and/or bioproduction.

The above workflow step can be at least one out of the group of media preparation, (complete) emptying, cleaning, maintaining, sterilizing, replacing and/or loading the first receptacle with media, preferably enriched media. "Media preparation" describes the act of composing the media to be used in the bioprocess, in particular the calculations of the specific component ratios and the actual manufacture of the media.

In a preferred embodiment, the workflow step may be at least partial, preferably complete, emptying of the first receptacle 4. In another example, the first receptacle 4 needs to be at least partially, preferably completely, cleaned. In a further example the workflow step may be the maintenance of the first receptacle 4, in order to ensure an optimal bioprocess, or a replacing of the first receptacle 4, in particular, if the receptacle 4, or in particular any receptacle of the bioprocess unit 3, is designed as a single-use device. Another possible workflow step could be the loading of the first receptacle 4 with media, in particular enriched media, as described above. For this procedure, the media needs to be filled into the receptacle, first. Any cultivation media can be used, preferably any enriched media, which comprises higher nutrient concentrations etc. Further preferably, these media do not constitute the limiting factor for cell cultivation and/or bioproduction. Here and preferably, and just as an example 4Cell^{®} CHO Media, NutriStem^{®} hPSC XF Medium, RoosterNourish^{™}-MSC, Gibco Cell Culture Medium, DMEM, IMDM, or the like might be used, depending on the bioprocess of choice. Further preferably, media that lead to optimal cell growth and proliferation rates, healthy physiology, morphology and/or proper gene expression are used.

In the further embodiments according to Fig. 1 and according to Fig. 2, the second culture environment B is provided by a second receptacle 16, which is separate from the first receptacle 4. This allows for a transfer of cells from the first receptacle 4 to a second receptacle 16. Preferably, when starting operation of the proposed bioprocess installation 1, the second receptacle 16 is free from cell broth of the first receptacle 4.

According to Fig. 1 and Fig. 2, the first receptacle 4 and/or the second receptacle 16 may comprise a feed line 17 for a controlled feeding of media during cultivation according to a predefined feeding profile. Such feeding profiles can be designed as pulse or continuous or a mixed feeding profile, wherein preferably the feeding profile is individually controllable and adjustable.

According to Fig. 1, the second receptacle 16 may be designed as a bioreactor. The term "bioreactor" in this context means any manufactured device or system that supports a biologically active environment by allowing monitoring and control of at least one parameter.

Additionally or alternatively, the second receptacle 16 is a passive receptacle 18 without a feed line 17 and/or without an electronic process control 2 (Fig. 2).

Hence, this second receptacle 16 can be designed as a passive receptacle 18 or an active receptacle, such as a bioreactor. "Passive receptacle" means a receptacle without a feed line 17 and/or without an electronic process control 2, which allows monitoring of at least one parameter within this passive receptacle 18. In contrast, "active receptacle" means a receptacle with a feed line 17 and/or with an electronic process control 2, hence allowing for monitoring and/or controlling of at least one parameter within this active receptacle such as a bioreactor.

In another embodiment, the cell broth in the second receptacle 16 is preferably transferred into the first receptacle 4 after an intermediate phase. "Intermediate phase" means a phase in which the cells are kept until spent media and cell debris is completely removed from the first receptacle 4.

Preferably, the cells are kept in this intermediate phase no longer until a predefined threshold with regard to certain vitality parameters are reached compared to corresponding vitality parameters of the cells in the first culture environment A. In particular, the cells are kept no longer in this intermediate phase until the cells reach a predefined threshold of at least one vitality parameter, including but not limited to viability, growth rate, productivity, oxygen uptake rate and/or biomass production rate. In another preferred embodiment, the at least one predefined vitality parameter, in particular viability, deviates less than 10 %, preferably less than 5 %, from the vitality parameter in the first culture.

In case the second receptacle 16 is a passive receptacle 18, as depicted in Fig. 2, the cell broth in the second receptacle 16 is preferably transferred to the first receptacle 4, not before a termination condition has been fulfilled with respect to the liquid level in the first receptacle 4. This means that, here and preferably, a specific liquid level in the first receptacle 4 must not be exceeded. The specific level can be individually defined according to the process requirements. The liquid level is preferably less than 10 % of the total receptacle volume, further preferably less than 5 % of the total receptacle volume, further preferably less than 1 % of the total receptacle volume. Further preferably the first receptacle 4 is entirely empty.

According to Fig. 1, the bioprocess installation 1 preferably comprises two or more bioprocess units 3 and the second receptacle 16 of each bioprocess unit 3 is the first receptacle 4 of a subsequent bioprocess unit 3 such that the bioprocess units 3 are cascaded. The term "Cascaded" means here a consecutive sequence of bioprocess units 3 that follow one after another. Hence, the proposed method for operating a bioprocess installation 1 can be intended for employing one single receptacle or a multitude of receptacles, in particular bioreactors.

The proposed method can be used with any type of cell culture system, in particular bioreactors. The method can also be used with bioreactors of any size, in particular lab bioreactors or production bioreactors, and/or, the bioreactor(s) can be made of plastic, in particular bioplastic, glass or stainless steel, or can be designed as single-use bioreactor(s). Further, the method can be used with stationary or portable bioreactors. In another aspect, the method allows for bioreactors, wherein the cell viability is very high because there is a reduction in the stresses on cells.

As indicated by Fig. 1 and Fig. 2, in backward operation, after transferring at least part of the discharged cells into the second culture environment B, a startup phase is preferably initiated in the second culture environment B. The purpose of this startup phase is to establish conditions for cell cultivation and/or bioproduction and is initiated in the second culture environment B prior to cell cultivation and/or bioproduction. Exemplary conditions can be a cell viability of at least 80 %, preferably of at least 90 %, further preferably of at least 95 %. Further exemplary conditions can be a viable cell concentration of at least 1x10⁷ cells per milliliter, preferably of at least 2x10⁷ cells per milliliter, further preferably of at least 3x10⁷ cells per milliliter, further preferably of at least 4x10⁷ cells per milliliter. In a preferred embodiment, in the startup phase, the discharged cells are grown to a cultivation stage used for cell proliferation and/or bioproduction, in particular the log phase.

"Cultivation stage" refers to the growth of cells in batch or fed-batch culture, which can be subdivided into four phases: Lag phase, log (also called "exponential") phase, stationary phase and death phase. The cultivation stage can be any cultivation stage suitable for the bioprocess of interest. Preferably, the log phase or stationary phase. The term "log phase" or "exponential phase" refers to a growth period characterized by cell doubling per time unit. The term "stationary phase" describes a situation in which growth rate and death rate are equal, caused by a growth-limiting factor such as the depletion of an essential nutrient, and/or the formation of an inhibitory product such as an organic acid.

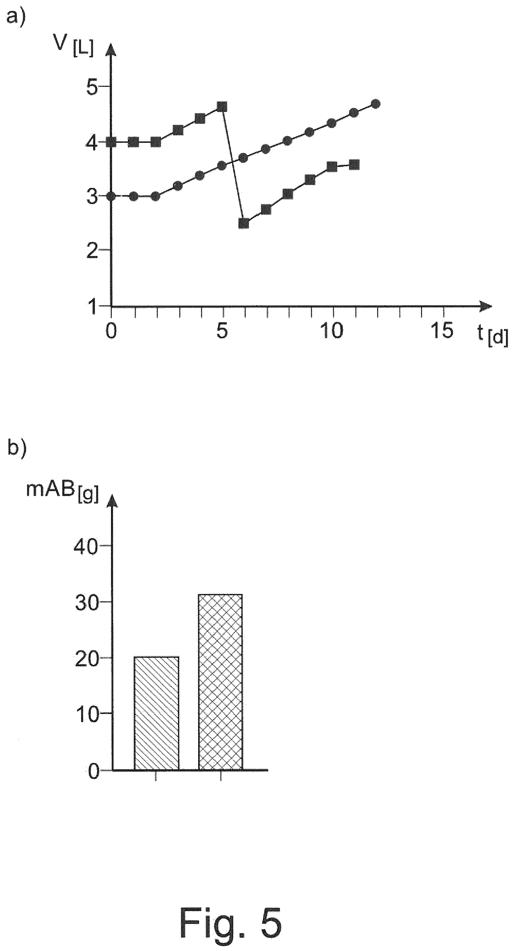

Proof of concept experiments have been conducted wherein a sterile glass vessel with a magnetic stir bar has been used as a second receptacle, wherein the harvested cells were transferred back into an emptied and cleaned bioreactor, which has been used as a first receptacle, according to the experimental setup shown in Fig. 2. A standard fed-batch process was conducted as a control experiment without the proposed method. The results are shown in Fig. 5. In both processes the same cell broth source, cultivation media components, process parameters and control strategy were used. The volumes over time of, on the one hand, the standard fed-batch process (first receptacle: a bioreactor, line with circles) and, on the other hand, the proposed method (first receptacle: a bioreactor, line with squares) may be taken from the graph of Fig. 5a. The increase of both process volumes' resulted from the addition of feed media to nourish the cells.

As shown in Fig. 5a, cells in media were cultivated in the bioreactor (first culture environment) for five days (line with squares) to a cell density of 2.5x10⁷ cells per milliliter and a total volume of 4.5 L. After five days of cultivation, the complete cell broth was intermediately harvested in forward operation of the centrifuge, leading to the separation of cells and, preferably cell free, supernatant. The separated supernatant from the intermediate harvest was transferred into a first glass vessel, while the intermediately harvested cells were washed in a cell washing step with 8.5 L of fresh enriched media transferred from a second glass vessel, which was pre-tempered overnight to 37 °C. These washed cells were collected in a third glass vessel (second receptacle) and exhibited a cell density of 3x10⁷ cells per milliliter.

After the bioreactor was completely harvested, the washed and concentrated cells were transferred from the third glass vessel back to the cleaned bioreactor (second culture environment).

Due to the concentration effect, a decrease in volume (2.5 L) in the bioreactor (first culture environment) occurred, as shown in Fig. 5a. After the intermediate harvest, cultivation in the bioreactor was continued until day 11, which is reflected by a corresponding, steady increase in volume.

As a result, the overall number of viable cells in the bioreactor (first culture environment) was determined and showed an increase by 33 % (not shown) compared to the standard fed-batch process. An increased viable cell concentration of 4.2x10⁷ cells per milliliter and a product recovery rate of at least 96 % were achieved (not shown).

Moreover, the proposed method led to an increase in product amount, here and preferably monoclonal antibody (mAB) amount. Looking at Fig. 5b, it becomes apparent that with a standard fed-batch process, an antibody amount of approx. 20 g (Fig. 5b, striped bar) was achieved, while > 30 g of antibody were produced using the proposed method (Fig. 5b, chequered bar). Hence, the proposed method led to an increase in product yield of approximately 50 % (Fig. 5b). A bioprocess is typically terminated as soon as at least one predefined termination criterion is fulfilled, such as a productivity threshold, cell viability threshold, and/or cell concentration threshold. Due to the higher yield, when using the proposed method, the cultivation time could be reduced by one day, in comparison to a standard fed-batch process (Fig. 5a).

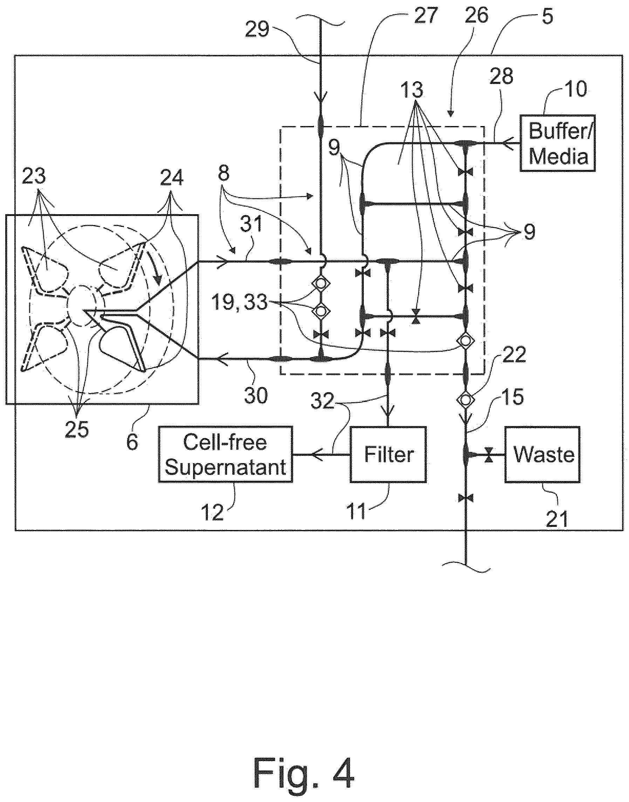

Here and preferably, in all embodiments according to Fig. 1 to 3, in forward operation for cell washing, washing liquid, preferably water, buffer or cultivation media, further preferably enriched media, coming from the buffer/media receptacle 10, is transferred to the centrifuge 6 via the liquid network 8 for cell washing, as indicated by Fig. 4. The water, buffer or cultivation media, preferably enriched media, used as washing liquid, can be either fresh or used or a mixture of both. In a preferred embodiment, the washing liquid is tempered to a predetermined temperature. Further preferably, the predetermined temperature is in fixed relation to the temperature of the cell broth source. In a preferred embodiment, the predetermined temperature can be adjusted according to the cells of choice, the bioprocess of choice and/or the product of choice. Preferably, the predetermined temperature is between 25 °C and 37 °C, further preferably, in case microbial or mammalian cells are used, between 35 °C and 37 °C, and/or, in case of insect, fish, or amphibian cells are used, between 25 °C and 28 °C.

Here and preferably, in all embodiments according to Fig. 1 to 3, in backward operation for cell discharging, discharging liquid, preferably water, buffer or cultivation media, further preferably enriched media, coming from the buffer/media receptacle 10, is transferred to the centrifuge 6 via the liquid network 8 for cell discharging, as indicated by Fig. 4. The water, buffer or cultivation media, preferably enriched media, used as discharging liquid, can be either fresh or used or a mixture of both. In a preferred embodiment, the discharging liquid is tempered to a predetermined temperature. Preferably, the predetermined temperature is in fixed relation to the temperature of the cell broth source. In a preferred embodiment, the predetermined temperature can be adjusted according to the cells of choice, the bioprocess of choice and/or the product of choice. Preferably, the predetermined temperature is between 25 °C and 37 °C, further preferably, in case microbial or mammalian cells are used for the bioprocess, between 35 °C and 37 °C, and/or, in case insect, fish, or amphibian cells are used, between 25 °C and 28 °C. The washing liquid and the discharging liquid can either be different or the same liquid.

The bioprocess installation 1, here and preferably, comprises a valve arrangement 14 with at least one valve 13, that allows to deactivate and activate at least one of the liquid lines 9, preferably by closing and opening the respective valve 13. Here and preferably, it is provided that the valve arrangement 14 is controlled by the electronic process control 2. The electronic process control 2 may activate or deactivate at least one of the liquid lines 9 via the valve arrangement 14 based on the sensor signals of the at least one sensor 19 of the sensor arrangement 20 and according to the respective control strategy.

Here, the term "deactivating" means, that liquid flow through the respective liquid line may be blocked by the respective valve. "Activating" means, accordingly, that liquid flow is allowed through the respective liquid line.

The valves 13 of the valve arrangement 14 are located within at least one of the respective liquid lines 9 and/or at one end of the respective liquid line(s) 9 to be activated and deactivated. The at least one valve 13 can be automatically selectively closed or opened during use. The individual valve(s) 13 of the valve arrangement 14 is/are controlled by the electronic process control 2.

The centrifuge 6 and/or the liquid pumping arrangement 7 and/or the valve arrangement 14 are preferably controlled by the electronic process control 2. The clarification setup 5, as can be seen in Fig.4, here and preferably comprises a sensor arrangement 20 with at least one sensor 19 for measuring properties of the liquid in at least one of the receptacles and/or in at least one of the liquid lines 9, which sensor arrangement 20 provides sensor signals to a process control, preferably the electronic process control 2. The process control of the sensor arrangement 20 may be designed as a centralized control unit, such as the electronic process control 2, or decentralized control unit. The sensor arrangement 20 provides sensor signals to the respective process control. Preferably, the sensor arrangement 20 measures at least one parameter out of the group of carbon-source concentration, nitrogen-source concentration, amino acids concentration, growth factors concentration, pH, temperature, oxygen concentration, carbon dioxide concentration, conductivity, pressure, biomass concentration, biomass production rate, product concentration, productivity and/or oxygen uptake rate.

In the preferred embodiments according to Fig. 1 to 3, the first receptacle 4 and/or the second receptacle 16 is/are provided as a bioreactor. Additionally or alternatively, a bioreactor process control, preferably the centralized or decentralized electronic process control 2, serves for monitoring and/or controlling at least one parameter in the first receptacle 4 and/or the second receptacle 16 out of the group of carbon-source concentration, nitrogen-source concentration, amino acids concentration, growth factors concentration, oxygen concentration, carbon dioxide concentration, pH, temperature, conductivity, pressure, biomass concentration, biomass production rate, product concentration, productivity, oxygen uptake rate and/or stirring speed. The bioreactor process control can be designed as a digital control unit (DCU) and/or as a multi fermenter control system (MFCS), or the like.

The term "Monitoring" means measurement and/or documentation of the, at least one, parameter measured by the sensor arrangement 20. The term "Controlling" means measurement, documentation and/or control of the at least one parameter measured by the sensor arrangement 20, and/or, is designed with or without a feedback control. The term "Feedback control" means a self-regulation of the system's activities based on the measurement of the at least one sensor 19 of the sensor arrangement 20.

As indicated by Fig. 4, the sensor arrangement 20 preferably comprises at least one biomass sensor 22 in the recycling line 15 and the clarification setup 5 comprises a waste receptacle 21, preferably designed as a waste vessel.

It is preferred, that the sensor arrangement 29 comprises a biomass sensor 22, preferably an optical biomass sensor, in the recycling line 15 for measuring an occurrence level of biomass, preferably the biomass concentration. During forward operation, depending on the measured occurrence level, preferably biomass concentration, the electronic process control 2 then deactivates or activates the feed line 17 and/or starts the washing step.

As an alternative or in addition, the sensor arrangement 20 comprises a supernatant sensor, preferably an optical supernatant sensor, for measuring an occurrence level of supernatant in the respective liquid line 9. During the forward operation, depending on the measured occurrence level of supernatant, here and preferably, the electronic process control 2 then deactivates or activates the respective liquid lines 9, preferably the recycling line 15, via the valve arrangement 14.

Here and preferably, in the backward operation of the centrifuge 6, depending on the sensor data of the biomass sensor 22 in the recycling line 15, the liquid, preferably the solid particles in discharging liquid, further preferably the cells in discharging liquid, is/are transferred to the second culture environment B or the waste receptacle 21 by the electronic process control 2 switching the valve arrangement 14 accordingly.

According to another, independent teaching, a bioprocess installation 1 is claimed as such, with an electronic process control 2 and at least one bioprocess unit 3. The bioprocess unit 3 comprises a cell broth source with a first receptacle 4 for cell broth including cultivation media and cells, establishing a culture environment A, B for cell cultivation and/or bioproduction. The bioprocess unit 3 further comprises a clarification setup 5 with a centrifuge 6 for the clarification of the cell broth by centrifugation, with a liquid pumping arrangement 7 assigned to the centrifuge 6 and with a liquid network 8 with a number of liquid lines 9 communicating with the liquid pumping arrangement 7, wherein out of a first culture environment A established by the first receptacle 4, the cell broth is transferred to the centrifuge 6 via the liquid network 8, which centrifuge 6 is operated in a forward operation for cell separation and/or cell washing and in a backward operation for cell discharging. Moreover, the bioprocess installation 1 comprises a valve arrangement 14, which allows to deactivate and activate at least one of the liquid lines 9. All explanations given before are fully applicable to this teaching.

It is essential, that the liquid network 8 comprises a recycling line 15 and that in the backward operation, at least part of the discharged cells may be transferred into a second culture environment B different from the first culture environment A via the recycling line 15 for subsequent cell cultivation and/or bioproduction.

The electronic process control 2 is preferably designed to perform the proposed method by controlling the centrifuge 6 and/or the liquid pumping arrangement 7 and/or the valve arrangement 14. The electronic process control 2 may be realized as a central unit controlling all or at least most of the components of the bioprocess installation 1. The electronic process control 2 may also be realized in a decentralized structure, comprising a number of decentralized units. In some embodiments, the at least one electronic process control(s) 2 direct(s) the opening and closing of the one or more valve 13, the flow rates of the one or more pump of the liquid pumping arrangement 7, the rotational speed of the rotor, either directly or via a motor, and/or the flow velocity of the fluid and/or particles from a cell broth source, such as a bioreactor.

Such an electronic process control 2 comprises for instance at least one digital control unit (DCU) and/or at least one multi fermenter control system (MFCS), which comprises a local processor unit and a local data storage itself. The MFCS also provides a centralized process management system, dispatching requests to the digital control unit. Additionally or alternatively, such an electronic process control 2 comprises preferably a computer, and/or a server, and/or a smartphone or the like. Here and preferably, the electronic process control 2 is individually adjustable and/or programmable and/or comprises at least one microprocessor, on which a software may be run.

As can be seen in the enlarged view in Fig. 4 showing of the clarification setup 5, here and preferably the centrifuge 6 comprises at least one centrifuge chamber 23 with a chamber inlet 24 and a chamber outlet 25.

The expression "chamber inlet" means that the liquid to be centrifuged enters the, at least one centrifuge chamber 23 via the chamber inlet 24. The expression "chamber outlet" means that the centrifuged liquid exits the at least one centrifuge chamber 23 via the chamber outlet 25. This is only to be understood as a definition of the fluid interface of the centrifuge chamber 23. In case the forward operation is switched to the backward operation, wherein the liquid pumping arrangement 7 inverts the direction of the fluid flow, the chamber inlet 24 may be used as a chamber outlet and the chamber outlet 25 may be used as a chamber inlet, respectively.

In the beginning of a forward operation it may be necessary, that remaining buffer and/or remaining supernatant in the centrifuge chamber 23 is flushed into the waste receptacle 21 via the liquid network 8, before the chamber outlet 25 is connected by valve switching to the filter arrangement 11. It may also be necessary, that in the beginning of the washing step, buffer and remaining supernatant in the centrifuge chamber 23 is being pumped to the filter arrangement 11, before the chamber outlet 26 is connected by valve switching to the waste receptacle 23 via the recycling line 10.

Here and preferably, the electronic process control 2 is automatically executing those steps sequentially, according to a certain, individually definable control strategy. In the easiest case, the control strategy includes the execution of the respective operations according to a fixed sequence in a fixed time pattern. However, the control strategy may well be based on sensor signals.

Generally, it may be provided, that the centrifuge 6 comprises a centrifuge chamber 23, which is designed as a single-use component, and/or, that the recycling line 15 is designed as a single-use component. As an alternative or in addition, it may be provided, that at least part of the liquid network 8 is designed as a single-use component. Further preferably, at least part of the filter arrangement 11 is designed as a single-use component. The single-use component is preferably made of plastic material at least partly, such that it may be realized with low effort. In a particularly preferred embodiment, the single-use component is at least partly made of a silicon material and/or polymer material and/or bioplastic.

According to another, independent teaching, an electronic process control of the bioprocess installation 1 is claimed as such. Again, reference is made to all explanations given before.

It is essential, that the electronic process control 2 is designed for performing the proposed method by controlling the centrifuge 6 and/or the liquid pumping arrangement 7 and/or the valve arrangement 14.

According to another, independent teaching, the use of a pre-mounted liquid network product 26, which comprises a structure of interconnected liquid lines 27, as at least part of the liquid network 8 of the proposed bioprocess installation 1, is claimed as such. Again, all explanations given before are fully applicable.

Here and preferably, and as can be seen in Fig. 1 to 3, the pre-mounted liquid network product 26 can be designed as two separate, preferably different, pre-mounted liquid network products 26, comprising two separate, preferably different, structures of interconnected liquid lines 27. One pre-mounted liquid network product 26 is preferably designed to support its use for supernatant clarification in the forward operation of the centrifuge 6 by providing at least one liquid line 9 of the liquid network 8, optimized for supernatant clarification, preferably optimized regarding a minimal liquid line distance, liquid network 8 and/or liquid lines 9. The second pre-mounted liquid network product 26 is designed to support its use for separation of cells and liquid in the backward operation of the centrifuge 6 by providing a liquid network 8 and liquid lines 9, optimized for the separation of cells and liquid, preferably optimized regarding a minimal liquid line distance, liquid network 8 and/or liquid lines 9.

Here and preferably, such a pre-mounted liquid network product 26 comprises at least one inlet buffer/media line 28, designed for a supply with buffer or media, one inlet cell broth line 29, designed for a supply with cell broth by the cell broth source, one inlet centrifuge line 30, designed for a supply with centrifuged cell broth via the chamber outlet 25, one outlet centrifuge line 31, designed for an outflow of liquid into the chamber inlet, one outlet supernatant line 32, designed for an outflow of supernatant towards the filter arrangement 11 and the supernatant reception 12 and one recycling line 15, designed for an outflow of liquid, in particular, discharged cells in discharging liquid, towards the waste receptacle 21 or the second culture environment B.

During the discharging step, the buffer may then be pumped from the buffer/media receptacle 10 to the chamber outlet 25 via an inlet buffer/media line 28, while the buffer or media including solid particles, preferably cell harvest, is flowing from the chamber inlet 24 towards the recycling line 15. During the discharging step, the liquid pressure and/or the liquid flow in the inlet buffer/media line 28 may be controlled by the liquid pumping arrangement 7.

In particular, the outlet supernatant line 32 as such may be provided as a single-use component. With this, it is particularly easy to guarantee the liquid tightness of the outlet supernatant line 32 without the risk of compromising the liquid tightness due to user errors during installation.

Here it is to be understood, that some of the liquid lines 9 may overlap, such that a respective liquid flow, originally assigned to a specific liquid line 9, may double-use at least one of the liquid lines 9, originally assigned to another liquid flow, at least along a certain liquid line 9 section. Accordingly, the respective liquid lines 9 do not have to be separate from each other along their complete extent. This is true for all other definitions of liquid lines 9 presented here and being part of the liquid network 8, which are each being provided by part of the liquid network 8.

Additionally or alternatively, the pre-mounted liquid network product comprises at least one, preferably at least three, further preferably exactly three, bubble sensor(s) 33. In order to distinguish liquid from the air, at least one of the bubble sensors 33 can be designed as an optical sensor and/or at least one of the bubble sensors 33 can be designed as ultrasonic sensor and/or at least one of the bubble sensors 33 can be designed as conductivity sensor. Depending on the sensor data of the at least one bubble sensor 33, the liquid is transferred to the respective liquid line(s) 9 and/or to the respective receptacle of interest.

In another preferred embodiment, in the enlarged view of the clarification setup 5 according to Fig. 4, the structure of interconnected liquid lines 27 of the pre-mounted liquid network product 26 provides at least the recycling line 15 of the at least one bioprocess unit 3.

In another preferred embodiment, the structure of interconnected liquid lines 27 of the pre-mounted liquid network product 26 is designed as a single-use component. All in all, it may be advantageous to provide all components, that are in direct contact with liquid, as single-use components. This would at least include all liquid lines 7, the at least one centrifuge chamber 23, as well as all filters of the filter arrangement 11. It may also include at least part of the valve arrangement 14 and/or at least part of the sensor arrangement 20. In particular, single-use sensors may be part of the sensor arrangement 20. Here and preferably, the pre-mounted liquid network product 26 comprises a sterile packaging for sterile housing of the structure of interconnected liquid lines 27.

## Claims

1. Method for operating a bioprocess installation (1) with an electronic process control (2) and at least one bioprocess unit (3), wherein the bioprocess unit (3) comprises a cell broth source with a first receptacle (4) for cell broth including cultivation media and cells, establishing a culture environment (A, B) for cell cultivation and/or bioproduction, wherein the bioprocess unit (3) comprises a clarification setup (5) with a centrifuge (6) for the clarification of the cell broth by centrifugation, with a liquid pumping arrangement (7) assigned to the centrifuge (6) and with a liquid network (8) with a number of liquid lines (9) communicating with the liquid pumping arrangement (7),
wherein out of a first culture environment (A) established by the first receptacle (4), the cell broth is transferred to the centrifuge (6) via the liquid network (8), which centrifuge (6) is operated in a forward operation for cell separation and/or cell washing and a backward operation for cell discharging,
**characterized in**
**that** the liquid network (8) comprises a recycling line (15) and that in the backward operation, at least part of the discharged cells is being transferred into a second culture environment (B) different from the first culture environment (A) via the recycling line (15) for subsequent cell cultivation and/or bioproduction.

2. Method according to claim 1, **characterized in that** the second culture environment (B) differs from the first culture environment (A) with respect to the structural entity establishing the respective culture environment (A, B), and/or, that the second culture environment (B) differs from the first culture environment (A) with respect to liquid properties, and/or, that the second culture environment (B) differs from the first culture environment (A) with respect to culture environment conditions.

3. Method according to claim 1 or 2, **characterized in that** the second culture environment (B) provides culture environment conditions thereby favoring cell growth and/or bioproduction, and/or, that the second culture environment (B) is the first culture environment (A), after having exchanged the cultivation media.

4. Method according to any one of the preceding claims, **characterized in that** the second culture environment (B) is provided by the first receptacle (4), which, for establishing the second culture environment (B), is being brought to a cell-free and/or a liquid-free state in a preparation phase, preferably, that the preparation phase consists of at least one workflow step to establish conditions for cell cultivation and/or bioproduction and that this workflow step can be at least one out of the group of media preparation, emptying, cleaning, maintaining, sterilizing, replacing and/or loading the first receptacle with media, preferably enriched media.

5. Method according to any one of the preceding claims, **characterized in that** the second culture environment (B) is provided by a second receptacle (16), which is separate from the first receptacle (4), preferably, that the second receptacle (16) is free from cell broth of the first receptacle (4).

6. Method according to any one of the preceding claims, **characterized in that** the first receptacle (4) and/or the second receptacle (16) comprise(s) a feed line (17) for a controlled feeding of media during cultivation according to a predefined feeding profile, and/or, that the second receptacle (16) is a passive receptacle (18) without a feed line (17) and/or without electronic process control (2).

7. Method according to claim 5 and, if so, to claim 6, **characterized in that** the cell broth in the second receptacle (16) is transferred into the first receptacle (4) after an intermediate phase, preferably, that the cells are kept no longer in the intermediate phase until the cells reach a predefined threshold of a given vitality parameter, preferably viability, wherein the given vitality parameter deviate less than 10% from the vitality parameter in the first culture environment (A).

8. Method according to claim 5 and, if the second receptacle (16) is a passive receptacle (18), also to claim 6 or 7, **characterized in that** the cell broth in the second receptacle (16) is transferred into the first receptacle (4) not before a termination condition has been fulfilled with respect to the liquid level in the first receptacle (4).

9. Method according to any one of the preceding claims, **characterized in that** the bioprocess installation (1) comprises two or more bioprocess units (3) and that the second receptacle (16) of each bioprocess unit (3) is the first receptacle (4) of a subsequent bioprocess unit (3) such that the bioprocess units (3) are cascaded.

10. Method according to any one of the preceding claims, **characterized in that** in the backward operation, after transferring at least part of the discharged cells into the second culture environment (B), a startup phase is initiated in the second culture environment (B) to establish conditions for cell cultivation and/or bioproduction, preferably, that in the startup phase, the discharged cells are grown to a cultivation stage used for bioproduction, in particular the exponential phase.

11. Method according to any one of the preceding claims, **characterized in that** in forward operation for cell washing, washing liquid, preferably buffer or cultivation media, is transferred to the centrifuge (6) via the liquid network (8), preferably, that the washing liquid is tempered to a predetermined temperature, further preferably, that the predetermined temperature is in fixed relation to the temperature of the cell broth source.

12. Method according to any one of the preceding claims, **characterized in that** in backward operation for cell discharging, discharging liquid, preferably buffer or cultivation media, is transferred to the centrifuge (6) via the liquid network (8), preferably, that the discharging liquid is tempered to a predetermined temperature, further preferably, that the predetermined temperature is in fixed relation to the temperature of the cell broth source.

13. Method according to any one of the preceding claims, **characterized in that** the bioprocess installation (1) comprises a valve arrangement (14), which allows to deactivate and activate at least one of the liquid lines (9).

14. Method according to any one of the preceding claims, **characterized in that** the centrifuge (6) and/or the liquid pumping arrangement (7) and/or the valve arrangement (14) are controlled by the electronic process control (2), preferably, that the bioprocess installation (1) comprises a sensor arrangement (20) for measuring properties of the liquid in at least one of the receptacles and/or in at least one of the liquid lines (9), which sensor arrangement (20) provides sensor signals to the electronic process control (2), preferably, that the sensor arrangement (20) measures at least one parameter out of the group of carbon-source concentration, nitrogen-source concentration, amino acids concentration, growth factors concentration, pH, temperature, oxygen concentration, carbon dioxide concentration, conductivity, pressure, biomass concentration, biomass production rate, product concentration, productivity and/or oxygen uptake rate.

15. Method according to any one of the preceding claims, **characterized in that** the first receptacle (4) and/or the second receptacle (16) is/are provided as a bioreactor, and/or, that a bioreactor process control serves for monitoring and/or controlling at least one parameter in the first receptacle (4) and/or the second receptacle (16) out of the group of carbon-source concentration, nitrogen-source concentration, amino acid concentration, growth factor concentration, oxygen concentration, carbon dioxide concentration, pH, temperature, conductivity, pressure, biomass concentration, biomass production rate, product concentration, productivity, oxygen uptake rate and/or stirring speed.

16. Method according to any one of the preceding claims, **characterized in that** the sensor arrangement (20) comprises a biomass sensor (22) in the recycling line (15), and that, in the backward operation of the centrifuge (6), depending on the sensor data of the biomass sensor (22), the liquid is transferred to the second culture environment (B) or the waste receptacle (21) by the electronic process control (2) switching the valve arrangement (14) accordingly.

17. Bioprocess installation with an electronic process control (2) and at least one bioprocess unit (3), wherein the bioprocess unit (3) comprises a cell broth source with a first receptacle (4) for cell broth including cultivation media and cells, establishing a culture environment (A, B) for cell cultivation and/or bioproduction, wherein the bioprocess unit (3) comprises a clarification setup (5) with a centrifuge (6) for the clarification of the cell broth by centrifugation, with a liquid pumping arrangement (7) assigned to the centrifuge (6) and with a liquid network (8) with a number of liquid lines (9) communicating with the liquid pumping arrangement (7),
wherein out of a first culture environment (A) established by the first receptacle (4), the cell broth is transferred to the centrifuge (6) via the liquid network (8), which centrifuge (6) is operated in a forward operation for cell separation and/or cell washing and a backward operation for cell discharging,
wherein the bioprocess installation (1) comprises a valve arrangement (14), which allows to deactivate and activate at least one of the liquid lines (9),
**characterized in**
**that** the liquid network (8) comprises a recycling line (15) and that in the backward operation, at least part of the discharged cells may be transferred into a second culture environment (B) different from the first culture environment (A) via the recycling line (15) for subsequent cell cultivation and/or bioproduction.

18. Bioprocess installation according to claim 17, **characterized in that** the electronic process control (2) is designed for performing the method according to any one of the claims 1 to 16 by controlling the centrifuge (6) and/or the liquid pumping arrangement (7) and/or the valve arrangement (14).

19. Bioprocess installation according to claim 17 or 18, **characterized in that** the centrifuge (6) comprises at least one centrifuge chamber (23) with a chamber inlet (24) and a chamber outlet (25), preferably, that the centrifuge (6) comprises a centrifuge chamber (23), which is designed as a single-use component, and/or, that the recycling line (15) is designed as a single-use component.

20. Electronic process control of the bioprocess installation (1) according to any one of the claims 17 to 19, **characterized in that** the electronic process control (2) is designed for performing the method according to any one of the claims 1 to 16 by controlling the centrifuge (6) and/or the liquid pumping arrangement (7) and/or the valve arrangement (14).

21. Use of a pre-mounted liquid network product (26), which comprises a structure of interconnected liquid lines (27), as at least part of the liquid network (8) of the bioprocess installation (1) according to any one of the claims 17 to 19.

22. Use according to claim 21, **characterized in that** the structure of interconnected liquid lines (27) of the pre-mounted liquid network product (26) provides at least the recycling line (15) of the at least one bioprocess unit (3).

23. Use according to claim 21 or 22, **characterized in that** the structure of interconnected liquid lines (27) of the pre-mounted liquid network product (26) is designed as a single-use component, preferably, that the pre-mounted liquid network product (26) comprises a sterile packaging for sterile housing of the structure of interconnected liquid lines (27).
